(19) Europäisches Patentamt
European Patent Office
Office européen des brevets

(11) Veröffentlichungsnummer: **0 127 605**
**A2**

(12) # EUROPÄISCHE PATENTANMELDUNG

(21) Anmeldenummer: **84890089.0**

(22) Anmeldetag: **17.05.84**

(51) Int. Cl.³: **A 61 K 35/16**

(30) Priorität: **31.05.83 AT 1983/83**

(43) Veröffentlichungstag der Anmeldung:
**05.12.84 Patentblatt 84/49**

(84) Benannte Vertragsstaaten:
**AT BE CH DE FR GB IT LI NL SE**

(71) Anmelder: **IMMUNO Aktiengesellschaft für chemisch-medizinische Produkte**
**Industriestrasse 72**
**A-1220 Wien(AT)**

(72) Erfinder: **Eibl, Johann, Dr.**
**Gustav Tschermakgasse 2**
**A-1180 Wien(AT)**

(72) Erfinder: **Wöber, Günter, Prof. Dr.**
**Carolusstrasse 23**
**A-2522 Oberwaltersdorf(AT)**

(74) Vertreter: **Wolfram, Gustav, Dipl.-Ing.**
**Schwindgasse 7 P.O. Box 205**
**A-1041 Wien(AT)**

(54) **Verfahren zur Herstellung von Präparationen aus menschlichem Blutplasma.**

(57) Es wird ein verfahren zur Herstellung von Präparationen aus menschlichem Blutplasma, wie Gerinnungsfaktorpräparationen, Globulinpräparationen, Plasmaenzymen und Plasmaenzyminhibitoren beschrieben, wobei der Plasmafraktion zur Inaktivierung etwa vorhandener vermehrungsfähiger Krankheitserreger Verbindungsgemische zugesetzt werden, die in freie Radikale zerfallen. Solche Verbindungen können Alkylperoxide, Acylperoxide, Peroxysäuren, Peroxyester und Peroxycarbonate sein. Es können Katalysatoren mitverwendet werden.

In Versuchen, bei welchen Hundehepatitisvirus zu den Plasmafraktionen zugesetzt wird, wird gezeigt, daß nach Behandlung der Plasmafraktionen mit freien Radikalen der Virustiter um mindestens vier Zehnerpotenzen abfällt, während die Aktivität der Plasmafraktionen im wesentlichen erhalten bleibt.

0 127 605 A2

# Verfahren zur Herstellung von Präparationen aus menschlichem Blutplasma

Die Erfindung betrifft ein Verfahren zur Herstellung von Präparationen aus menschlichem Blutplasma, wie Gerinnungsfaktorpräparationen, Globulinpräparationen, Plasmaenzymen und Plasmaenzyminhibitoren.

Trotz sorgfältiger Überprüfung von Blutspendern ist das Risiko, nach der Übertragung von Blut oder Blutderivaten an mikrobiellen Infektionen zu erkranken, nicht gering. Es gibt zwar mehrfach Bemühungen, Krankheitserreger in Blutpräparationen, wie Plasmafraktionen, zu inaktivieren, doch läßt die Sicherheit dieser Verfahren zu wünschen übrig.

So ist es aus der veröffentlichten europäischen Patentanmeldung 0 018 561 bekannt, Gerinnungsfaktoren (darunter Faktor VIII) enthaltende Präparationen, die praktisch fibrinogenfrei sind, gegen Hitzeeinwirkung zu stabilisieren, indem der Lösung der Gerinnungsfaktoren Aminosäure und ein Mono-, Oligosaccharid oder Zuckeralkohol zugesetzt wird. Nach einer solchen Behandlung kann die Präparation 10 Stunden bei 60°C erhitzt werden, wobei es bekannt ist, daß eine solche Behandlung bei Humanalbumin eine Inaktivierung von Hepatitisviren bewirkt. Das bekannte Verfahren hat den Nachteil, daß die schützenden Komponenten in hohen Konzentrationen - nämlich bis zur Löslichkeitsgrenze - zugesetzt werden müssen und daß die Ausbeute an den Gerinnungsfaktoren nicht zufriedenstellend ist.

In der veröffentlichten europäischen Patentanmeldung 0 035 204 ist weiters ein Verfahren zur Herstellung

einer - u.a. den Faktor VIII enthaltenden - Proteinkomposition beschrieben, welches Verfahren darin besteht, daß die Komposition mit einem Polyol gemischt und die Mischung erhitzt wird, während einer Dauer, die ausreicht, um die Proteinkomposition zu pasteurisieren.

Die bisher vorgeschlagenen Verfahren waren im wesentlichen darauf gerichtet, Präparationen herzustellen, die frei waren von Hepatitis-B-Virus, was durch Nachweis der Nicht-Infizierbarkeit von Schimpansen mit der entsprechenden Präparation überprüft werden konnte, doch hatte und hat man keine Kenntnis über das sonstige Wirkungsspektrum der vorgeschlagenen Maßnahmen. Es hat sich z.B. gezeigt, daß die in den angezogenen Literaturstellen empfohlenen Maßnahmen gegen das Hundehepatitisvirus als Modellvirus gänzlich wirkungslos waren. Es bestehen somit bei der Übertragung von Blutplasmapräparationen weiterhin Infektionsrisken, z.B. im Zusammenhang mit Non-A-non-B-Hepatitis-Virus oder mit unbekannten, derzeit nicht nachweisbaren Viren.

Die Erfindung bezweckt die Vermeidung der geschilderten Nachteile und Schwierigkeiten und stellt sich die Aufgabe, ein Verfahren zur Inaktivierung von vermehrungsfähigen Krankheitserregern in Blutplasmapräparationen zu schaffen, welches nicht nur gegen Hepatitisviren, sondern gegen ein weites Spektrum von Krankheitserregern einschließlich unbekannter Viren wirksam ist, wobei gleichzeitig die Erhaltung der biologischen Aktivität und der molekularen Integrität der wertvollen Blutprodukte angestrebt wird.

Die Erfindung, die diese Aufgabe löst, besteht darin, daß Blutplasmafraktionen mit einem freien Radikal behandelt werden. Unter einem "freien

Radikal" werden heute meist sehr kurzlebige, valenzmäßig ungesättigte und daher reaktionsfähige, anorganische und organische Verbindungen verstanden, die einsame Elektronen bzw. eine ungerade Zahl von Elektronen im Molekül aufweisen.

Es ist zwar bereits in der DE-A - 1 617 500 vorgeschlagen worden, zur Konservierung von Blutplasma ein Acetalperoxid zu verwenden, wobei bei Aufbewahrung in Abwesenheit von Sauerstoff die Lösung keimfrei bleibt, jedoch ist dieses Verfahren zur Inaktivierung von Plasmafraktionen gegenüber einem breiten Spektrum von Viren nicht geeignet, weil das als Hauptbestandteil im Vollplasma vorhandene Albumin einen Inhibitionseffekt gegenüber der bakteriziden Aktivität von Peroxidase aufweist (vgl. "Bactericidal Acitivity of Eosinophil Peroxidase" von E.C. Jong et.al. in The Journal of Immunology, Vol. 124, No. 3, March 1980, 1378-1382).

Es ist auch schon vorgeschlagen worden, wässerige Virensuspensionen durch anodische Oxidation (vgl. H. Mahnel, Zbl. Bakt. Hyg. I. Abt. Orig. B 166, 542-557 (1978), Virusinaktivierung in Wasser durch anodische Oxidation) oder durch Kontakt mit freien Radikalen zu behandeln (vgl. Belding M.E., S.J. Klebanoff und C.G. Ray, Science 167, 195-196, 1970, Peroxidase-mediated virudical systems), um eine Inaktivierung zu erreichen, doch erfolgte diese Behandlung an Viruskulturen als solchen, d.h. in Abwesenheit von Blutprodukten. Da Blutprodukte jedoch, wie bekannt ist, manchmal eine Schutzwirkung auf die Viren ausüben, konnte aus den Ergebnissen von Belding et al. die Lösung der vorliegenden Aufgabe nicht abgeleitet werden.

Beispielsweise können im erfindungsgemäßen Verfahren freie Radikale in Reaktionsmischungen

$$\cdot O_2 \xrightarrow{+1\,e^{\ominus}} {}^{*}O_2^{\ominus} \xrightarrow[+2\,H^{\oplus}]{+1\,e^{\ominus}} H_2O_2$$

Sauerstoff — Superoxid-Radikalanion — Wasserstoffperoxid

$$H_2O_2 \xrightarrow{+1\,e^{\ominus}} {}^{*}OH + (+\,OH^{\ominus}) \xrightarrow{+1\,e^{\ominus}} H_2O$$

Hydroxylradikal — Wasser

gebildet werden.

Weitere Beispiele sind die Bildung von freien Radikalen aus Alkylperoxiden, Acylperoxiden, Peroxysäuren, Peroxyestern und Peroxycarbonaten.

Alkylperoxide sind organische Verbindungen der allgemeinen Formel

$$R'\!-\!\!(OOR)x \quad ,$$

in der R und R' Alkyl- oder substituierte Alkylgruppen bedeuten. In Alkylhydroperoxiden ist R'=H und x=1 oder 2. In üblichen, monofunktionellen Alkylperoxiden bedeuten x = 1, R' = H oder Alkyl, z.B.:

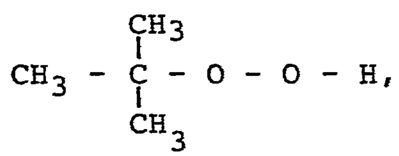

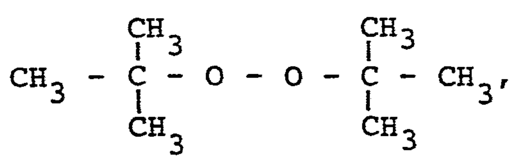

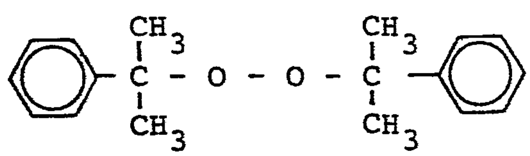

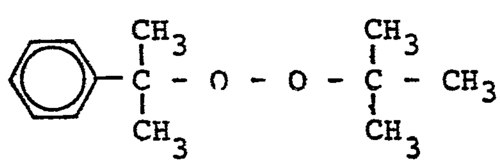

In den üblichen bifunktionellen Alkylperoxiden bedeuten z.B. x = 2, R = t-Butyl und R' = t-Alkyldiradikal, wie

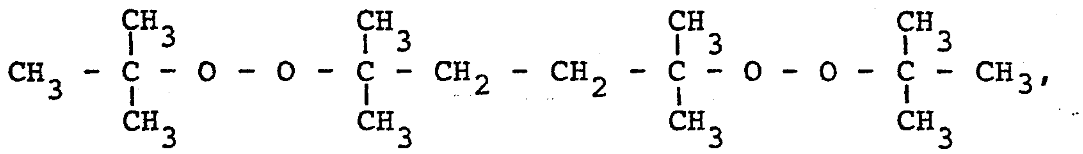

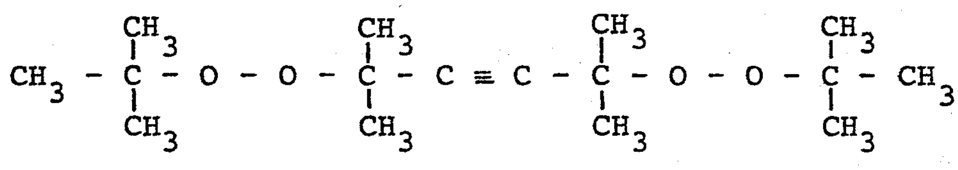

Alkylperoxide erleiden einen homolytischen Zerfall in zwei freie Radikale:

$$R - O - O - H \longrightarrow R - O^{*} + {}^{*}OH$$
$$R - O - O - R' \longrightarrow R - O^{*} + R' - O^{*}$$

t-Alkoxy-Radikale können eine weitere Spaltung erleiden, die zu Ketonen und Alkylradikalen führt:

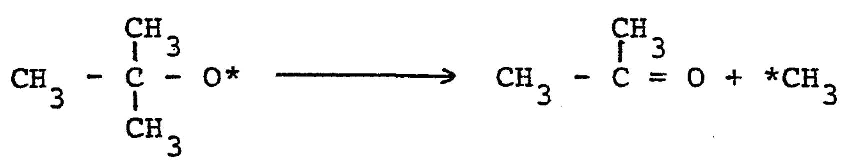

Diacylperoxide haben folgende allgemeine Formel:

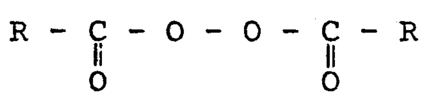

Übliche Diacylperoxide sind z.B.

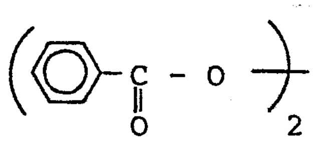 ,

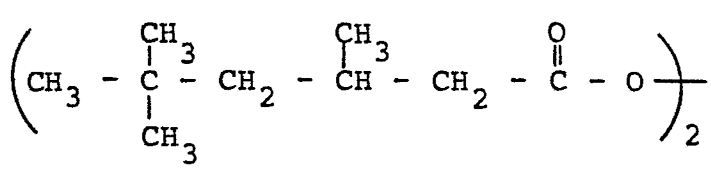 ,

0127605

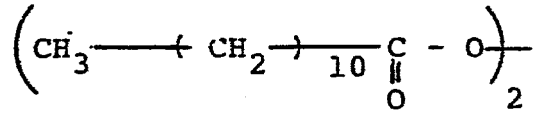

In verdünnten Lösungen zerfallen Diacylperoxide unter Bildung freier Radikale:

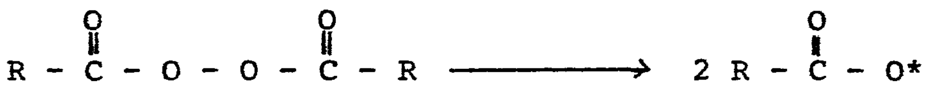

Peroxysäuren und Peroxyester

Die wichtigsten Gruppen üblicher Persäuren und Perester sind: Alkylperoxyester von Monocarbonsäuren mit der allgemeinen Formel:

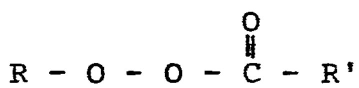

R = H : Peroxysäure

z.B.:

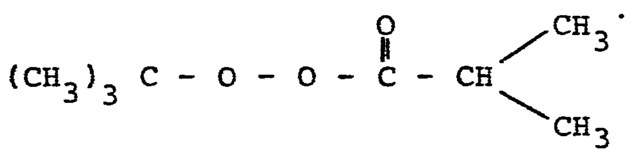

,

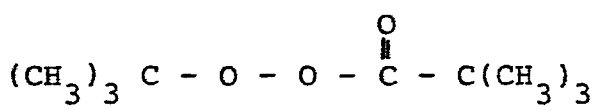

,

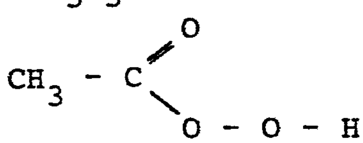

,

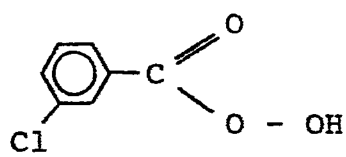

Mono- und Dialkylperoxyester und -peroxysäuren mit der allgemeinen Formel

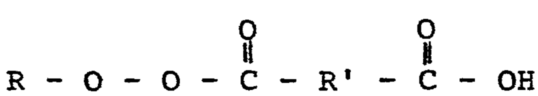

R = H : Peroxysäure

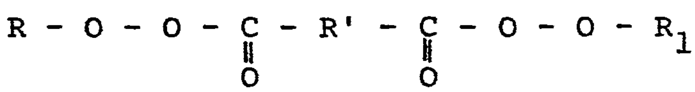

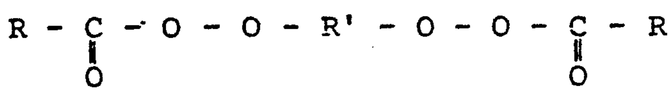

z.B.:

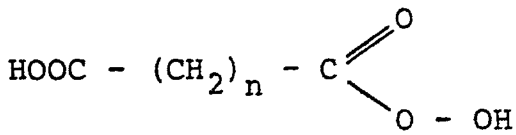

n = 2 oder 3,

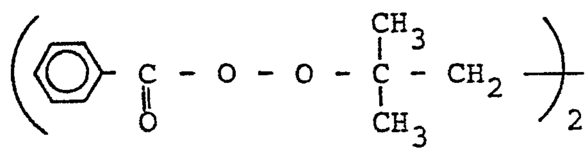

,

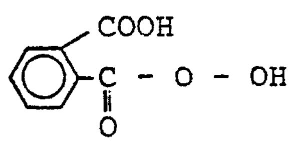

,

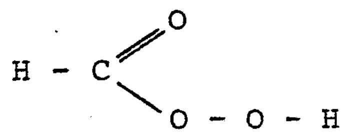

Bei der homolytischen Spaltung von Persäuren oder Perestern entstehen freie Radikale. Diperester reagieren in analoger Weise.

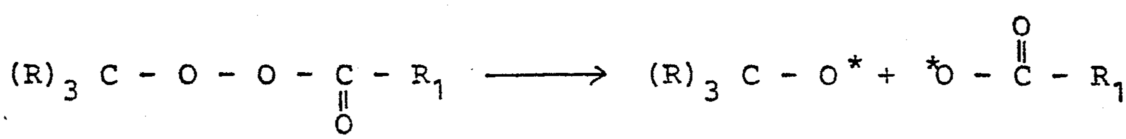

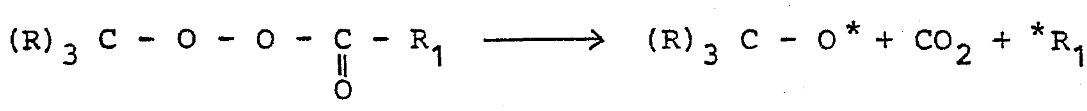

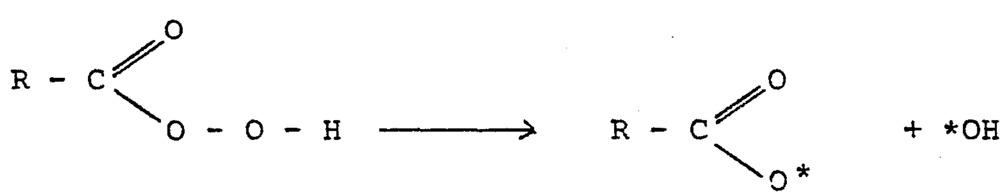

Peroxycarbonate sind eine Klasse organischer Peroxyverbindungen der allgemeinen Formel:

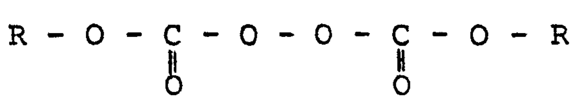

,

in welcher R eine primäre oder sekundäre Alkyl-, Cycloalkyl-, Aralkyl- oder Arylgruppe bedeuten kann, z.B.:

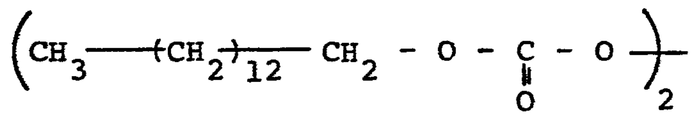

Die homolytische Spaltung mit nachfolgender Decarboxylierung unter Bildung freier Radikale ist die wichtigste Reaktion der Percarbonate:

1. 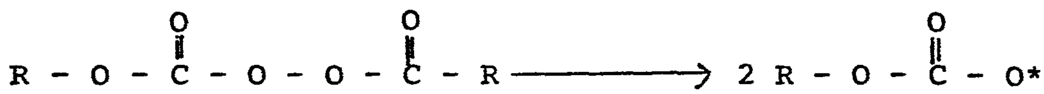

2. 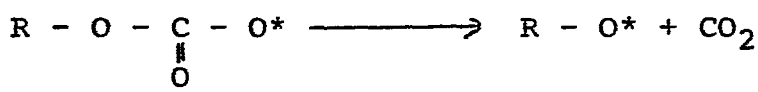

Die erfindungsgemäß zu behandelnden Plasmafraktionen können in flüssiger oder fester, z.B. lyophilisierter Form vorliegen.

Gemäß einer bevorzugten Ausführungsform wird der Plasmafraktion eine Verbindung zugesetzt, die in freie Radikale zerfällt, wobei vorteilhaft Peressigsäure zugesetzt wird, die in die freien Radikale *Acetyl und *OH zerfällt.

Zweckmäßig wird ein unter Freisetzung eines freien Radikals reagierendes Gemisch, wie $H_2O_2$ und ein Reduktionsmittel zugesetzt.

Aus $H_2O_2$ entsteht durch Reduktion das Hydroxylradikal. Als Reduktionsmittel kann beispielsweise Ascorbinsäure, Dihydroxymaleinsäure oder das Superoxid-Radikalanion dienen. Diese Reaktion wird durch Übergangsmetallionen oder deren Chelatkomplexe katalysiert:

$$[Y]_{red} + H_2O_2 \xrightarrow[EDTA]{Fe^{3\oplus}} {}^{*}OH + OH^{\ominus} + [Y]_{OX}$$

Das reaktive Gemisch aus Superoxid-Radikal und $H_2O_2$ kann auch in situ durch univalente Reduktion von $O_2$ mittels Xanthin-Oxidase, gefolgt von spontaner Disproportionierung des Superoxid-Radikals zu $H_2O_2$ (und $O_2$) erzeugt werden.

Vorteilhaft wird dem reagierenden Gemisch ein Katalysator zugesetzt, wobei als Katalysator ein Übergangsmetallion oder Metallionenkoordinationskomplexe, wie Äthylendiamintetraacetat, verwendet werden.

Zweckmäßig werden dem Substrat $H_2O_2$, ein Halogenid oder Pseudohalogenid oder eine Mischung derselben und ein biologischer Katalysator, wie eine Peroxidase, zugesetzt.

Gemäß einer bevorzugten Ausführungsform wird die Behandlung in einem Temperaturbereich von 0 bis 121°C während einer Dauer von 0,1 sec bis 24 Stunden, insbesondere 0,1 sec bis 1 Stunde, bei einem pH-Wert von 5 bis 9 durchgeführt.

Vorteilhaft wird zwecks Erreichung einer Breitbandwirkung die Behandlung der Plasmafraktion mit einem freien Radikal in Kombination mit anderen Virus-Inaktivierungsbehandlungen, insbesondere einer Hitzebehandlung, vorgenommen, wobei diese Behandlung nacheinander in der gleichen oder in verschiedenen Stufen des Fraktionierungsprozesses durchgeführt wird.

Zweckmäßig wird der Plasmafraktion ein Radikalfänger, wie Zuckeralkohole oder Thioharnstoff, zugesetzt.

Nach einer weiteren Ausführungsform der Erfindung wird die Plasmafraktion zur Zerstörung von etwa vorhandenen restlichen freien Radikalen bei Temperaturen von 40 bis 100°C während einer Dauer von 1 Stunde bis 5 Tage nachbehandelt.

Die Wirkung der erfindungsgemäßen Inaktivierungsmaßnahmen wird in den folgenden Modellversuchen veranschaulicht.

Versuch 1:

Es wurde eine Faktor VIII-Präparation gemäß der in der US-A - 3 973 002 beschriebenen Methode bereitet. Der erhaltenen Lösung mit einem Gehalt an Faktor VIII von 3 bis 4 E/ml wurde eine Hundehepatitis-Virus-Suspension mit einem Virustiter von $10^{5,1}$ $TCID_{50}/0,1$ ml zugesetzt. Die Mischung wurde auf einen pH-Wert von 8 eingestellt und eine wässerige Lösung von Peressigsäure in solcher Menge zugesetzt, daß eine Endkonzentration von 0,1 mM erreicht wurde. Die Mischung wurde während 30 min bei 37°C gehalten. Nach dieser Behandlung wurde der Virustiter bestimmt und festgestellt, daß dieser auf weniger als $10^1$ abgenommen hatte. Eine Bestimmung der verbliebenen Faktor VIII-Aktivität ergab 80 % Restaktivität.

Versuch 2:

Es wurde eine Prothrombin-Präparation gemäß der in Vox. Sang. 33: 3750 p. 37-50 (1977) beschriebenen Methode aus menschlichem Plasma nach Abtrennung der Gerinnungsfaktoren II, IX und X bereitet. Der erhaltenen Lösung mit einem Gehalt an 70 E Faktor II, 52 E Faktor IX und 61 E Faktor X/ml wurde eine Hundehepatitis-Virus-Suspension mit einem Virustiter von $10^{4,9}$ $TCID_{50}/0,1$ ml zugesetzt. Die Mischung wurde auf einen pH-Wert von 8 eingestellt und eine Lösung von Metachlorperoxybenzoesäure in solcher Menge zugesetzt, daß eine Endkonzentration von 0,1 mM erreicht wurde. Die Mischung wurde während 30 min bei 45°C gehalten. Nach dieser Behandlung wurde der Virustiter bestimmt und festgestellt, daß dieser auf weniger als $10^1$ abgenommen hatte. Eine Bestimmung der verbliebenen Faktor X-Aktivität ergab 86 % Restaktivität.

Versuch 3:

Der Versuch Nr. 2 wurde wiederholt, jedoch statt Metachlorperoxybenzoesäure unter Zusatz einer das freie Radikal *OH abgebenden Mischung von $H_2O_2$ und Ascorbinsäure in einer Konzentration von je 10 mM in der Mischung und Zugabe eines Katalysators $Fe^{++}$ und Äthylendiamintetraacetat in einer Konzentration von je 10 µm. Bei diesem Versuch wurde ebenfalls eine Abnahme des Virustiters von $10^5$ auf weniger als $10^1$ festgestellt, wobei eine Restaktivität an Faktor X von 25 % behalten wurde.

Weitere Versuche wurden durchgeführt, wobei auch andere Testviren verwendet wurden. Es wurde festgestellt, daß das erfindungsgemäße Verfahren in etwa gleicher Weise wirksam ist gegen Coxsackie-Virus $B_4$ und andere Viren.

Versuch 4:

Es wurde eine Faktor VIII-Präparation gemäß der in der US-A - 3 973 002 beschriebenen Methode bereitet. Der erhaltenen Lösung mit einem Gehalt an Faktor VIII von 3 bis 4 E/ml wurde eine Hundehepatitis-Virus-Suspension mit einem Virustiter von $10^{4,7}$ $TCID_{50}$/0,1 ml zugesetzt. Die Mischung wurde auf einen pH-Wert von 5 eingestellt und wässerige Lösungen von $H_2O_2$ und Kaliumjodid in solchen Mengen zugesetzt, daß Endkonzentrationen von 100 µm und 30 µm im Test erreicht wurden. Nach Zusatz von 2 I.U. Lactoperoxidase (Sigma) wurden während einer Reaktionsdauer von 30 min bei 37°C Jodradikale gebildet. Nach dieser Behandlung wurde der Virustiter bestimmt und festgestellt, daß dieser auf weniger als $10^1$ abgenommen hatte. Eine Bestimmung der verbliebenen Faktor VIII-Aktivität ergab 60 % Restaktivität.

Patentansprüche:

1. Verfahren zur Herstellung von Präparationen aus menschlichem Blutplasma, dadurch gekennzeichnet, daß Plasmafraktionen zur Inaktivierung etwa vorhandener vermehrungsfähiger Krankheitserreger mit einem freien Radikal behandelt werden.

2. Verfahren nach Anspruch 1, dadurch gekennzeichnet, daß der Plasmafraktion eine Verbindung in wässeriger oder nicht wässeriger Phase oder in Gemischen daraus zugesetzt wird, die in freie Radikale zerfällt.

3. Verfahren nach Anspruch 2, dadurch gekennzeichnet, daß Peressigsäure zugesetzt wird, die in die freien Radikale *Acetyl und *OH zerfällt.

4. Verfahren nach Anspruch 1, dadurch gekennzeichnet, daß der Plasmafraktion ein unter Freisetzung eines freien Radikals reagierendes Gemisch, wie $H_2O_2$ und ein Reduktionsmittel, wie Ascorbinsäure, zugesetzt wird.

5. Verfahren nach Anspruch 4, dadurch gekennzeichnet, daß dem reagierenden Gemisch ein Katalysator zugesetzt wird.

6. Verfahren nach Anspruch 5, dadurch gekennzeichnet, daß als Katalysator ein Übergangsmetallion oder Metallionenkoordinationskomplexe, wie Äthylendiamintetraacetat, verwendet werden.

7. Verfahren nach Anspruch 1, dadurch gekennzeichnet, daß der Plasmafraktion $H_2O_2$, ein Halogenid oder Pseudohalogenid oder eine Mischung derselben und ein biologischer Katalysator, wie eine Peroxidase, zugesetzt werden.

8. Verfahren nach den Ansprüchen 1 bis 7, dadurch gekennzeichnet, daß die Behandlung in einem Temperaturbereich von 0 bis 121°C durchgeführt wird.

9. Verfahren nach den Ansprüchen 1 bis 8, dadurch gekennzeichnet, daß die Behandlung während einer Dauer von 0,1 sec bis 24 Stunden, insbesondere 0,1 sec bis 1 Stunde, durchgeführt wird.

10. Verfahren nach den Ansprüchen 1 bis 9, dadurch gekennzeichnet, daß die Behandlung bei einem pH-Wert von 5 bis 9 durchgeführt wird.

11. Verfahren nach den Ansprüchen 1 bis 10, dadurch gekennzeichnet, daß zwecks Erreichung einer Breitbandwirkung die Behandlung der Plasmafraktion mit einem freien Radikal in Kombination mit anderen Virus-Inaktivierungsbehandlungen, insbesondere einer Hitzebehandlung, vorgenommen wird, wobei diese Behandlung nacheinander in der gleichen oder in verschiedenen Stufen des Fraktionierungsprozesses durchgeführt wird.

12. Verfahren nach den Ansprüchen 1 bis 11, dadurch gekennzeichnet, daß der Plasmafraktion ein Radikalfänger, wie Zuckeralkohole oder Thioharnstoff, zugesetzt wird.

13. Verfahren nach den Ansprüchen 1 bis 12, dadurch gekennzeichnet, daß die Plasmafraktion zur Zerstörung von etwa vorhandenen restlichen freien Radikalen bei Temperaturen von 40 bis 100°C während einer Dauer von 1 Stunde bis 5 Tage nachbehandelt wird.